(19) **Europäisches Patentamt / European Patent Office / Office européen des brevets**

(11) **EP 1 802 759 B1**

(12) # EUROPEAN PATENT SPECIFICATION

(45) Date of publication and mention of the grant of the patent:
**18.05.2016 Bulletin 2016/20**

(51) Int Cl.:
*C12N 15/79* *(2006.01)*   *C12N 15/86* *(2006.01)*
*C12N 5/16* *(2006.01)*   *C12N 15/09* *(2006.01)*

(21) Application number: **05856432.9**

(22) Date of filing: **05.10.2005**

(86) International application number:
**PCT/KR2005/003274**

(87) International publication number:
**WO 2006/080682 (03.08.2006 Gazette 2006/31)**

(54) **CONSTRUCT OF SWINE VESICULAR DISEASE VIRUS LIKE PARTICLE USING GENETIC ENGINEERING AND THE METHOD FOR MANUFACTURING THEREOF**

KONSTRUKT AUS EINEM VESIKULÄRSCHWEINEVIRUSÄHNLICHEN TEILCHEN DURCH GENETISCHES ENGINEERING UND HERSTELLUNGSVERFAHREN DAFÜR

PRODUIT DE RECOMBINAISON DE PARTICULES VIROIDES DE LA MALADIE VÉSICULEUSE DU PORC PAR GÉNIE GÉNÉTIQUE ET PROCÉDÉ PERMETTANT DE PRÉPARER CELUI-CI

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **20.10.2004 KR 20040083994**

(43) Date of publication of application:
**04.07.2007 Bulletin 2007/27**

(73) Proprietor: **Republic of Korea**
**Anyang-si, Gyeonggi-do 430-824 (KR)**

(72) Inventors:
 • **CHOI, Kang Seuk**
  **Yeongdeungpo-gu,**
  **Seoul 150-797 (KR)**
 • **KO, Young Joon**
  **Anyang-si,**
  **Gyeonggi-do 430-822 (KR)**
 • **NAH, Jin Ju**
  **Gyeonggi-do 430-839 (KR)**
 • **JO, Nam In**
  **Gangnam-gu,**
  **Seoul 135-010 (KR)**

(74) Representative: **Ahner, Philippe et al**
 **BREVALEX**
 **95, rue d'Amsterdam**
 **75378 Paris Cedex 8 (FR)**

(56) References cited:
**US-B1- 6 200 576   US-B1- 6 531 136**

 • **KO YOUNG-JOON ET AL: "Noninfectious virus-like particle antigen for detection of swine vesicular disease virus antibodies in pigs by enzyme-linked immunosorbent assay." CLINICAL AND DIAGNOSTIC LABORATORY IMMUNOLOGY AUG 2005, vol. 12, no. 8, August 2005 (2005-08), pages 922-929, XP002525920 ISSN: 1071-412X**
 • **JIMENEZ-CLAVERO MIGUEL A ET AL: "Molecular cloning, expression and immunological analysis of the capsid precursor polypeptide (P1) from swine vesicular disease virus" VIRUS RESEARCH, vol. 57, no. 2, October 1998 (1998-10), pages 163-170, XP002525921 ISSN: 0168-1702**
 • **HU Y.C. ET AL.: 'Formation of enterovirus-like particle aggregates by recombin ant baculoviruses co-expressing P1 and 3CD in insect cells' BIOTECHNOL. LETT. vol. 25, no. 12, June 2003, pages 919 - 925, XP008119794**
 • **BORREGO B. ET AL.: 'Mapping of linear epitopes on the capsid proteins of swine vesicular disease virus using monoclonal antibodies' J. GEN. VIROL. vol. 83, no. PT 6, June 2002, pages 1387 - 1395, XP008119795**
 • **ZHANG G. ET AL. J. GEN. VIROL. vol. 74, no. PT 5, May 1993, pages 845 - 853, XP008119796**

EP 1 802 759 B1

• LINDBERG A.M. ET AL. J. GEN. VIROL. vol. 84, no. PT 5, May 2003, pages 1223 - 1235, XP008119797

**Description**

[Technical Field]

The present invention relates to a swine vesicular disease virus-like particle prepared by a gene recombination technique and a preparation method thereof. More specifically, the present invention relates to a method for preparing a swine vesicular disease virus-like particle, wherein the structural protein precursor (P1) gene and 3CD protease gene of total genes of swine vesicular disease virus are inserted simultaneously into a baculovirus expression vector and are expressed in insect cells, so that the expressed 3CD protease enzyme digests the structural protein precursor into individual proteins which then form the capsid of swine vesicular disease virus by an self-assembly process.

[Background Art]

Swine vesicular disease is a highly contagious viral disease of pigs, characterized by vesicles on the coronary bands, heels of the feet, and occasionally on the lips, tongue, snout and teats after an incubation period of 2-7 days. Affected pigs recovered from the disease in 2-3 weeks. The swine vesicular disease is classified as a list A disease by the Office International des Epizooties (OIE), because its symptoms are clinically indistinguishable from those caused by foot-and-mouth disease virus.

When it was first observed in Italy in 1966, it was recognized as porcine enterovirus infection, after which it was termed "swine vesicular disease" after appearance in Great Britain in 1972. The etiology of swine vesicular disease is a swine vesicular disease virus which is a porcine variant caused by the infection of pigs with human Coxsackievirus B5. It belongs to the 'Enterovirus' genus of the *'Picornaviridae'* family according to microbiological classification, and has only one serotype. The swine vesicular disease virus is a (+) single-chain RNA virus consisting of about 7,400 bases and is highly stable in the surrounding environment unlike foot-and-mouse disease virus so that it is not influenced at a pH range of 2.5-12.

The swine vesicular disease virus has four main structural proteins (VP1, VP2, VP3 and VP4), and also has non-structural proteins, such as 2A, 2B, 2C, 3A, 3B, 3C and 3D, which are known to be expressed mainly in infected cells so as to perform subsidiary functions in intracellular replication of the virus.

As a method for detecting swine vesicular disease virus antibodies, the virus neutralization test has been recognized officially as a gold standard test method, but was unsuitable to test a large amount of samples due to disadvantages in that it requires a test period of about 2-3 days and cell culture facilities. As an alternative to solve such disadvantages, the enzyme-linked immunosorbent assay was developed [Journal of Virological Methods, (1995) 52: 155-167] . However, in the enzyme-linked immunosorbent assay which is currently available internationally to detect the swine vesicular disease virus antibodies, an inactivated swine vesicular disease virus is used as a diagnostic antigen. In this case, because live virus should be produced by using cell culture in the step of preparing the diagnostic antigen, a biosafety level (BSL) 3 laboratory must be required to prevent laboratory accidents (release of pathogens, etc). Also, because a carcinogenic substance, such as binary ethyleneimine, is used in a process of inactivating the virus, factors harmful to workers are latent. For these reasons, the method using the inactivated swine vesicular disease virus can be regarded as a highly hazardous and complicated operation.

In an attempt to solve the above-described problems, studies to develop recombinant protein antigens and to substitute the inactivated viral antigen with the safe recombinant antigens are ongoing worldwide. However, a recombinant protein antigen, which can sufficiently substitute for the inactivated swine vesicular disease antigen, is not yet developed.

For example, there is an example where a structural protein precursor (P1) was expressed in *E. coli* and attempted to use as a diagnostic antigen [Virus Research (1998) 57: 163-170]. However, because the swine vesicular disease virus has a densely-packed icosahedral arrangement of 60 protomers, each consisting of 4 proteins (VP1, VP2, VP3 and VP), most of antigenic sites are conformation dependent, whereas, in the case of the P1 recombinant protein only expressed in *E. coli,* the conformational structure of the viral antigenic sites could not be formed. This is demonstrated from the fact that a virus-neutralizing antibody was not detected in the serum of pigs immunized with the P1 precursor.

[Disclosure]

[Technical Problem]

The construction of a swine vesicular disease virus-like particle to be achieved according to the present invention has not been attempted on the swine vesicular disease virus. However, virus-like particles have been studied on some viruses, such as poliovirus and enterovirus, members of the Enterovirus genus to which the swine vesicular disease virus belongs. Also, virus-like particles have been produced either by infecting insect cells with a recombinant baculovirus inserted with the full-length genes (6.6-kb size including structural proteins and non-structural proteins) of poliovirus [Journal of General Virology (1989) 70: 1453-1463], or by co-infecting insect cells with each of three recombinant baculoviruses inserted with structural proteins VP0 (VP4+VP2), VP3 and VP1-coding genes [Virology (1993) 192: 512-524], or by co-infecting insect cells with baculoviruses inserted with each of the P1 and 3CD-encoding genes of enterobirus 71 [Biotechnology Letters (2003) 25: 919-925].

However, as a result of close analysis of the examples for the construct of the enterovirus like particle, in the case of using the recombinant baculovirus inserted with the full-length virus genes, the metabolism in the insect cells was overly loaded, thus limiting the expression of the genes. Also, in the case of either using three recombinant baculoviruses inserted with each of structural proteins VP0 (VP4+VP2), VP3 and VP1-coding genes or using recombinant baculoviruses inserted with each of the P1 and 3CD-coding genes of enterovirus 71, the same cells must be infected with at least two kinds of baculoviruses. For this reason, because it was difficult to standardize a suitable level and rate of infection with each of baculoviruses, the stable production of the virus-like particles could not be expected and the production of the virus-like particles was also very low.

[Technical Solution]

The present inventors have found from inspecting current models for recombinant antigen that an antigen obtained either by the individual preparation of each of four structural proteins forming the capsid of a swine vesicular disease virus or by the expression of only the structural protein precursor (P1) is not possible to mimic the conformational immunodominant antigenic sites. Accordingly, it was concluded that a usable recombinant protein antigen must have a structure morphologically similar to the structure of the native swine vesicular disease virus, and for this purpose, it is a preferable approach to construct a recombinant protein in the form of a virus-like particle.

Based on this fact, the present inventors have found that, when a recombinant baculovirus inserted with both the structural protein precursor P1 and 3CD protease genes is constructed and used, the overload problem caused by the size of inserted genes can be minimized and the standardization of the amount of its infection into insect cells can be achieved in a very easy manner, and when insect cells are infected with the baculovirus, the P1 protein expressed in the insect cells can be digested with the 3D protease protein expressed simultaneously with the P1 protein so as to produce a virus-like particle, thereby completing the present invention.

Accordingly, it is an object of the present invention to provide a swine vesicular disease virus-like particle assembled by recombinant proteins expressed by infecting insect cells with a recombinant baculovirus inserted with both the structural protein precursor P1 and 3CD protease genes of swine vesicular disease virus, as well as a preparation method thereof.

[Description of Drawings]

FIG. 1 is a schematic diagram of a baculovirus expression vector inserted with both the structural protein precursor P1 and 3CD genes of swine vesicular disease virus.
FIG. 2 is a schematic diagram showing a strategy for constructing a swine vesicular disease virus-like particle.
FIG. 3 is an electron microphotograph of swine vesicular disease virus-like particles expressed in a gene recombinant baculovirus.
FIG. 4 shows the results of Western blotting analysis for a swine vesicular virus particle.

Lane 1: Protein marker;
Lane 2: Reactivity of swine vesicular disease virus-like particle to guinea pig antiserum;
Lane 3: Reactivity of inactivated swine vesicular disease virus to guinea pig antiserum;
Lane 4: Reactivity of swine vesicular disease virus-like particle to pig antiserum; and
Lane 5: Reactivity of inactivated swine vesicular disease virus-like particle to pig antiserum.

[Best Mode]

To achieve the above object, the present invention provides a swine vesicular disease virus-like particle assembled by recombinant proteins expressed by infecting insect cells with a recombinant baculovirus constructed using a baculovirus expression vector prepared by the simultaneous insertion of the structural precursor P1 gene and 3CD protease gene of swine vesicular disease virus.

The recombinant baculovirus expression vector (hereinafter, referred to as "pFastDual/P1/3CD") is characterized by containing both the structural protein precursor P1 gene and 3CD protease gene of swine vesicular disease virus.

The recombinant baculovirus (hereinafter, referred to as "Bacmid/P1/3CD") according to the present invention is characterized by containing both the structural protein precursor P1 gene and 3CD protease gene of swine vesicular disease virus.

The structural protein precursor P1 gene of swine vesicular disease virus used in the present invention is derived from the UKG/27/72 strain isolated in Great Britain in 1972, and its gene sequence (SEQ ID N0: 1) was registered in the National Center for Biotechnology Information (NCBI) under accession number X54521, and a protein (SEQ ID N0: 2) encoded by the gene consists of 851 amino acids.

The 3CD protease gene (SEQ ID N0: 3) of swine vesicular virus gene used in the present invention is derived from the UKG/27/72 strain isolated in Great Britain in 1972, and its gene sequence (SEQ ID NO: 3) was registered in the National Center for Biotechnology Information (NCBI) under accession number X54521, and a protein (SEQ ID N0: 4) encoded by the gene consists of 645 amino acids.

The swine vesicular disease virus-like particle according to the present invention was successfully developed for the first time in the world in the research field of swine vesicular disease virus, and can be used as a new diagnostic antigen which will substitute for the inactivated virus antigen currently used in ELISAs for detection of antibodies to swine vesicular disease virus.

Also, the swine vesicular disease virus-like particle according to the present invention has an advantage in that it can be prepared without handling of any hazardous chemical during the preparation of the swine vesicular disease virus-like particle. In addition, it can also be used as an antigen for preventive vaccines against swine vesicular disease because its antigenicity is highly similar to that of the native virus antigen.

In another aspect, the present invention provides a method for preparing a swine vesicular virus-like particle using a gene recombination technique, the method comprising the steps of: (1) amplifying the structural protein precursor P1 gene and 3CD protease gene of swine vesicular disease virus by polymerase chain reaction (PCR); (2) inserting the P1 gene into a baculovirus expression vector (pFastBacDual) so as to prepare pFastBacDual/P1; (3) inserting the 3CD gene into the pFastBacDual/P1 inserted with the P1 gene so as to prepare pFastBacDual/P1/3CD; (4) transforming a baculovirus shuttle vector gene (Bacmid)-containing cell (DH10Bac) with the pFastBacDual/P1/3CD containing the P1 gene and the 3CD gene so as to prepare recombinant baculovirus gene Bacmid/P1/3CD; (5) transfecting insect cells with the Bacmid/P1/3CD gene; (6) subjecting the supernatant of the transfected insect cells to a plaque assay so as to isolate a pure recombinant Baculovirus; and (7) infecting insect cells with the recombinant baculovirus so as to obtain a swine vesicular disease virus-like particle.

The experiment procedures, reagents and reaction conditions, which can be used in the method for preparing the recombinant baculovirus, are generally known in the prior art. The inventive method for preparing the virus-like particle can be used in a wide range of applications, since it can likewise be applied for other enteroviruses having the same genetic structure.

The expression vector used in the present invention is not limited to the above-described expression vector, because other kinds of expression baculovirus expression vectors as dual expression vectors may also be used in the present invention.

The swine vesicular disease virus-like particle prepared according to the above-described method is obtained at an expression level of more than 1.0 mg/liter, indicating that the inventive method overcomes the problem of low expression levels, which is a problem shown in the construction of the poliovirus virus-like particle according to the prior art. Also, in a test for the comparison of titer between the supernatant of insect cells infected with recombinant baculovirus and the supernatant of host cells (IBRS-2) infected with swine vesicular disease virus, the swine vesicular disease virus-like particle prepared using the recombinant baculovirus showed a titer equal to that of the inactivated swine vesicular disease virus. Thus, the inventive virus-like particle can be developed into a subunit vaccine.

[Mode for Invention]

Hereinafter, the present invention will be described in more detail by examples. It will, however, be obvious to those skilled in the art that these examples are not construed to limit the scope of the present invention, and various modifications, additions and substitutions are possible within the scope of the claims.

Example 1: Extraction of RNA from swine vesicular disease virus

The swine vesicular disease virus (UKG/27/72) strain isolated in Great Britain in 1972 was inoculated into IBRS-2 cells (National Veterinary Research and Quarantine Service, Korea), and then, total RNA was extracted from the supernatant of the culture using the RNeasy RNA extraction kit (Qiagen) according to the manufacturer's manual.

Example 2: Synthesis, amplification and cloning of P1 gene cDNA of swine vesicular disease virus

To synthesize the P1 gene cDNA of swine vesicular disease virus, the total RNA extracted in Example 1 and a reverse primer of SEQ ID N0: 6 were added into the tube of an RT premix kit (Bioneer) where a first strand cDNA was then synthesized according to the manufacturer's manual. Then, the first strand cDNA, a forward primer of DEQ ID N0: 5 and a reverse primer of SEQ ID N0: 6 were thermally treated at 95°C for 10 min in the tube of a PCR premix kit (Bioneer) for polymerase chain reaction (PCR). Then, the cDNA was amplified in a PCR amplifier (PE 2400; Perkin Elmer) for 30 cycles of denaturation at 94°C for 1 min, annealing at 55°C for 1 min and extension at 72°C for 2 min, and finally extended at 72°C for 5 min.

To perform the above PCR reaction, the following PCR primers were synthesized based on the swine vesicular disease virus P1 gene sequence registered in the National Center for Biotechnology Information (NCBI, Genbank) under accession number X54521:

Forward primer: 5'-*GGATCC*ATGGGAGCTCAAGTGTCAAC-3' (SEQ ID N0: 5)
Reverse primer: 5'-*AAGCTT*AAGTGGTTTTCATGGTTGTTA-3' (SEQ ID N0: 6)

To construct a baculovirus expression vector below, the forward primer of SEQ ID N0: 7 and the reverse primer of SEQ ID N0: 8 were intentionally inserted into the *BamHI* restriction enzymes site upstream of the 5'-terminal ATG, and the *HindIII* restriction enzyme site upstream of the 5'.-terminal stop codon, respectively. The P1 gene cDNA thus amplified was cloned into a pGEM-T easy vector (Promega, USA) so as to construct "pGEMT/P1", and the base sequence of the inserted gene was comparatively analyzed with the known base sequence of SEQ ID N0: 1 using an automatic sequencer (ABI 377, USA) to examine if the P1 gene cDNA was correctly inserted.

Example 3: Synthesis, amplification and cloning of 3CD gene cDNA of swine vesicular disease virus

To synthesize the 3CD gene cDNA of swine vesicular disease virus, the total RNA extracted in Example 1 and a reverse primer of SEQ ID N0: 8 were added into the tube of an RT premix kit (Bioneer) where a first strand cDNA was then synthesized according to the manufacturer's manual. Then, the first strand cDNA, a forward primer of SEQ ID N0: 7 and a reverse primer of SEQ ID N0: 8 were thermally treated at 95°C for 10 minutes in the tube of a PCR. premix kit (Bioneer) for polymerase chain reaction (PCR). Then, the cDNA was PCR-amplified in a PCR amplifier (PE 2400; Perkin Elmer) for 30 cycles of denaturation at 94°C for 1 min, annealing at 55°C for 1 min and extension at 72°C for 2 min and finally extended at 72°C for 5 min.

To perform the above PCR reaction, the following PCR primers were synthesized based on the swine vesicular disease virus P1 gene sequence registered in the National Center for Biotechnology Information (NCBI, Genbank) under accession number X54521:

Forward primer: 5'-GCC*CTCGAG*ATGGGTCCAGCGTTTGAGTTC-3' (SEQ ID N0: 7)
Reverse primer: 5'-GCTC*GGTACC*TAAAAGGAGTCCAACCACTT-3' (SEQ ID N0: 8)

To construct a baculovirus expression vector below, the forward primer of SEQ ID NO: 7 and the reverse prime of SEQ ID N0: 8 were intentionally inserted into the *XhoI* restriction enzyme site upstream of the 5'-terminal ATG and the *kpnI* restriction enzymes site upstream of the 5'-terminal stop codon, respectively. The 3CD gene cDNA thus amplified was cloned into a pGEM-Teasy vector (Promega, USA) so as to construct "pGEMT/3CD", and the base sequence of the inserted gene was comparatively analyzed with the known base sequence of SEQ ID N0: 3 using an automatic sequencer (ABI 377, USA) to examine if the 3CD gene was correctly inserted.

Example 4: Construction of recombinant expression vector inserted with P1 gene of swine vesicular disease virus

The pGEMT/P1 vector prepared in Example 2 was digested with *BamHI* and *HindIII* restriction enzymes so as to extract the P1 DNA fragment. Meanwhile, a pFastBacDual™ vector (Invitrogen, USA) was digested with the same restriction enzymes, and the P1 DNA fragment was inserted downstream of the polyhedrin promoter ($P_{PH}$)-containing gene site of the vector, thus constructing recombinant baculovirus expression vector pFastBacDual/P1 inserted with the P1 gene of swine vesicular disease virus.

Example 5: Construction of recombinant expression vector inserted with both P1 and 3CD genes of swine vesicular disease virus

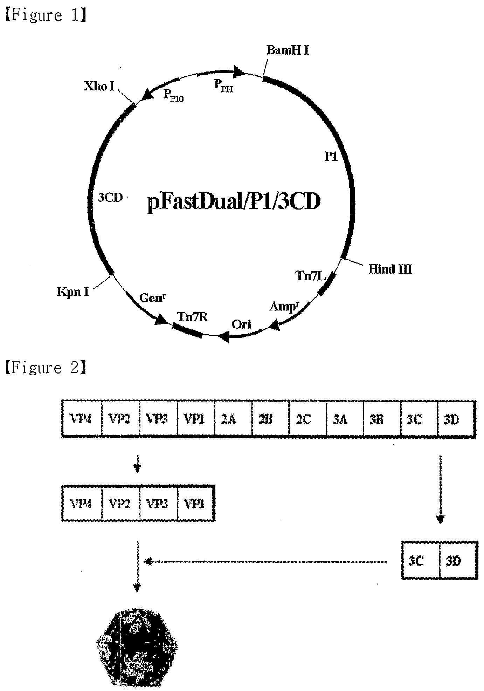

The recombinant baculovirus expression vector pFastBacDual/P1 prepared in Example 4 was digested with *XhoI* and Kpn*I* restriction enzymes, and the pGEMT/3CD constructed in Example 3 was digested with the same restriction enzymes and then inserted downstream of the P10 promoter ($P_{p10}$)-containing gene site of the pFastBacDual/P1, thus constructing recombinant baculovirus expression vector pFastBacDual/P1/3CD inserted with both the P1 and 3CD genes of swine vesicular disease virus. FIG. 1 shows the structure of the recombinant baculovirus expression vector (pFast-BacDual/P1/3CD) according to the present invention.

Example 6: Expression in insect cells and preparation of recombinant baculovirus

A recombinant baculovirus (bacmid/P1/3CD) according to the present invention was constructed using a Bac-to-Bac baculovirus expression system (Invitrogen, USA) in the following manner. The pFastDual/P1/3CD vector constructed in Example 5 was transfected into a cell (DH10Bac) containing a baculovirus gene (bacmid) so as to construct recombinant baculovirus DNA (Bacmid/P1/3CD).

A process for preparing a recombinant baculovirus using the recombinant baculovirus DNA (bacmid/P1/3CD) is as follows. A solution of 1 $\mu$g of a purified Bacmid/P1/3CD gene in 100 $\mu\ell$ of serum-free grace medium was mixed with a solution of 6 $\mu$g of CellFectin (Invitrogen) dissolved in 100 $\mu\ell$ of serum-free grace medium, and the mixture solution was left to stand at room temperature for 30 minutes. Meanwhile, a *Spodoptera frugiperda* (hereinafter, referred to as "Sf9") cell line (GibcoBRL) pre-cultured in a 35-mm-diameter cell culture Petri dish well to a concentration of $2\times10^6$ cells was washed one time with a serum-free Grace's medium which was then removed.

To the solution mixture, 0.8 ml of serum-free grace medium was added. Then, the mixture was added to the Sf9 cells, after which the resulting cells were transfected by culturing at 27°C for 5 hours. After removing the supernatant of the cultured cells, 2 ml of a fresh grace medium containing 10% fetal bovine serum was added thereto and the cells were cultured at 27°C for 6 days while the appearance or non-appearance of cytopathic effect (CPE) was observed. The culture supernatant of the transfected cells was collected and subjected to a plaque assay so as to isolate a pure recombinant 4 baculovirus. Namely, $3\times10^4$ Sf9 cells were applied in a monolayer to the 35-mm-diameter cell culture Petri dish well and then inoculated with 1 ml of a 10-fold dilution of the above-collected supernatant, and the cells were sensitized at 27°C for 1 hour. After removing the supernatant, the cell culture medium was overlaid with a grace medium containing 1% low-melting point agarose so as to form a layer thereon, and the resulting cells were cultured for 8 days at 27°C to confirm the formation of plaques. A few plaques were collected and transfected into Sf9 cells, and the cells were cultured for 4 days and observed for the appearance or non-appearance of CPE. The collected cell homogenate was subjected to the ELISA assay so as to select a recombinant baculovirus (Bacmid/P1/3CD) reacted with a swine vesicular disease virus-specific monoclonal antibody.

Example 7: Reparation of swine vesicular disease virus-like particle using recombinant baculovirus

First, the Sf9 insect cells monolayer-cultured in a 150-cm$^2$ cell culture flask were infected with the recombinant baculovirus (Bacmid/P1/3CD prepared in Example 6, to a concentration of 0.1 M0I (multiplicity of infection) at 27 °C for 1 hour. The infected cells were collected and placed into a spinner culture flask containing 200 ml of Sf9 insect cells having a cell concentration of $2.0\times10^6$/m$\ell$, followed by culture at 27C for 4 days. The spinner-cultured infected cells were collected and centrifuged at 500xg for 20 minutes, after which the supernatant and the infected cells were collected separately. The supernatant was used as a diagnostic antigen by itself, and the infected cells were subjected to a series of the following procedures so as to prepare a diagnostic antigen. Namely, the infected cells were suspended in 0.01 M phospahte buffered saline (PBS) containing 1% Triton X-100 at a ratio of 1 (cell culture medium): 20 (PBS) and then disrupted with a sonicator. Then, the culture medium was centrifuged at 500xg for 20 minutes so as to collect only the supernatant for use as swine vesicular disease virus-like particles.

Example 8: Purification and electron microphotography of swine vesicular disease virus-like particle

The Sf9 cell culture medium infected with the recombinant baculovirus in the spinner culture flask in the same manner as in Example 7 was collected. Then, the collected culture medium was frozen and thawed three times repeatedly so as to disrupt the infected cells, followed by centrifugation at 8,000xg for 40 minutes to collect the supernatant. To the collected supernatant, 23 g/l of 0.5M NaCl and 75 g/l of 7.5% PEG were added, and the mixture was allowed to react at 4°C for 16 hours. Then, the reaction mixture was centrifuged at 8,000xg for 40 minutes so as to collect only the pellets. Then, the pellets were suspended in PBS at a ratio of 1 (culture medium): 50 (PBS) and dialyzed two times. The concentrated sample was centrifuged at 3,000xg for 5 minutes so as to collect the supernatant. The supernatant was subjected to ultracentrifugation using cesium chloride (CsCl) gradient so as to purify swine vesicular disease virus-like particle. More specifically, into the ultracentrifugation SW41 Ti tube of a ultracentrifuge (Beckman, USA), 5.5 ml of 40% CsCl and 5.5 ml of 5% CsCl were placed and then, to the upper layer of the CsCl, 0.5 ml of the above-concentrated supernatant was carefully added, and the mixture was centrifuged using an SW41 Ti rotor (Beckmann, USA) at 35,000 rpm for 6 hours. The fraction showing a white band was recovered and subjected to electron microscopy in the following manner.

Twenty $\mu\ell$ volume of the supernatant was placed into a parafilm, on which a grid was then floated for 5 minutes.

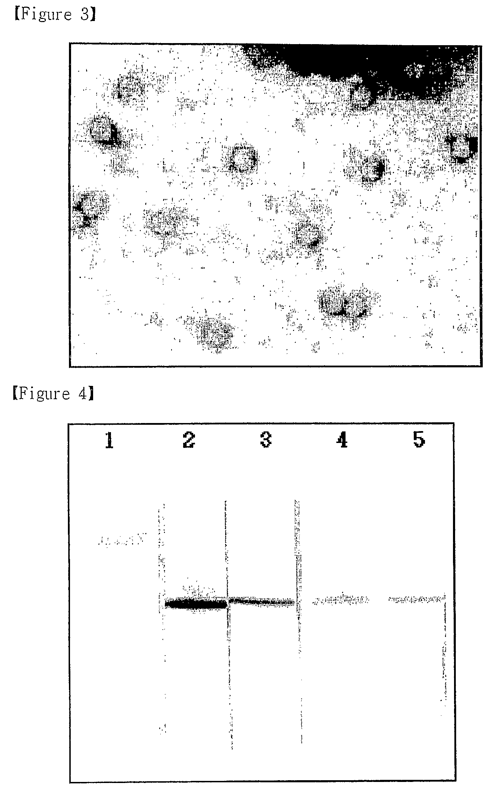

The grid was picked up with a pincette and immersed in 1% uranyl acetate, followed by drying at room temperature for 40 minutes. Then, the grid was observed for swine vesicular disease virus-like particles using an electron microscope, and the results are shown in FIG. 3. It was found that the swine vesicular disease virus-like particles prepared in the present invention had a size of 25-27 nm similar to 25-30 nm which is the size of the native virus containing nucleic acids.

Test Example 1: Analysis of swine vesicular disease virus-like particle by Western blotting

Proteins of the swine vesicular disease virus-like particle purified in Example 8 were electrophoretically separated on NuPAGE Novex Bis-Tris gels (Invitrogen, USA), and then were transferred from the gels onto a nitrocellulose membrane. The nitrocellulose membrane was treated with a blocking solution containing 5% skimmed milk, and then allowed to react with membranes to which guinea pig positive serum and pig positive serum have been transferred, respectively. The membrane was washed with a PBS containing 0.05% Tween 20 (PBST) and allowed to react with various alkaline phosphatase-conjugated secondary antibodies (KPL, USA) for 1 hour. Following washing step using PBST, speicific antigen-antibody reaction on the membrane was visualized by addition with a solution of BCIP (5-bromo-4-chloro-3-indolylphosphate)/NBT (nitroblue tetrazolium), and the results are shown in FIG. 4.

As shown in FIG. 4, the swine vesicular disease antibody-positive guinea pig sera (lanes 2 and 3) and the swine vesicular disease antibody-positive pig sera (lanes 4 and 5) showed reactivity to the swine vesicular disease virus-like particle (lanes 2 and 4) in the same pattern as the swine vesicular disease virus particle (lanes 3 and 5). This suggests that the virus-like particle observed with the electron microscope as described above is a swine vesicular disease virus-like particle having the same component as that of the native swine vesicular disease virus.

Test Example 2: Evaluation of antigenicity of swine vesicular disease virus-like particle

Using competitive ELISA assays (c-ELISA assays), the swine vesicular disease virus-like particle according to the present invention was evaluated for its antigenicity in comparison with that of the inactivated swine vesicular disease virus antigen of the prior art.

1) Evaluation of antigenicity by competitive ELISA assays

Swine vesicular virus monoclonal antibody 5B7 (swine vesicular disease virus-neutralizing antibody) was coated onto ELISA plates at 4°C overnight. On the ELISA plates, each of the swine vesicular disease virus and the swine vesicular disease virus-like particle was added and then allowed to react with the antibody at 37°C for 1 hour. Following washing step using PBST, pig sera (including swine vesicular disease standard-positive serum) were added thereto and allowed to react at 37°C for 1 hour. To the reaction mixtures, horseradish peroxidase-conjugated monoclonal antibody 5B7 (the same as used in the above coating) was added and allowed to react at 37°C for 1 hour. Following washing step using PBST, an ortho-phenylenediamine (OPD) solution as a color-developing agent was added thereto for 10 minutes and then allowed to react for 10 minutes, after which the reactions were terminated with a sulfuric acid solution. Then, visualized reaction was measured for the absorbance at 492nm. The competitive reactivity to an antigen of each serum was calculated as percent inhibition (PI) of the absorbance of a serum sample/monoclonal antibody mixture, relative to the absorbance of a monoclonal antibody, as shown in the following formula 1, and the results are shown in Table 1 below.

[Formula 1]

$$PI = 100 - (\text{absorbance of serum/monoclonal antibody mixture})/(\text{absorbance of monoclonal antibody}) \times 100$$

[Table 1]

| Test sera | Competitive ELISA assay (PI) | |
|---|---|---|
| | Inventive virus-like particles | Swine vesicular disease virus |
| Standard serum 1 | 4.0 | 1.5 |
| Standard serum 2 | 98.9 | 99.5 |
| Standard serum 3 | 91.9 | 93.7 |

(continued)

| Test sera | Competitive ELISA assay (PI) | |
|---|---|---|
| | Inventive virus-like particles | Swine vesicular disease virus |
| Standard serum 4 | 79 | 71.7 |
| Standard serum 5 | 91 | 85 |
| Standard serum 6 | 95.3 | 94.5 |
| Negative serum 7 | 20.3 | 10.3 |
| Negative serum 8 | 20.1 | 13.7 |
| ※ Reactivity correlation coefficient between two antigens: 0.997 (99.7%) | | |

As can be seen in Table 1 above, the antigenicity correlation coefficient between the inventive swine vesicular disease virus-like particle and the inactivated swine vesicular disease virus antigen was 0.997, indicating that the similarity between the two antigens is significantly high.

Also, the inventive virus-like particle simultaneously showed capturing and detection by the same monoclonal antibody, like the case of the inactivated swine vesicular disease virus. This suggests that the inventive virus-like particle has at least two exposed antigenic sites recognized by the same monoclonal antibody, which indirectly demonstrates the formation of a virus-like particle.

Because the monoclonal antibody 5B7 used above is an antibody having neutralizing activity against the swine vesicular disease virus, the above results suggest that the inventive swine vesicular disease virus-like particle has the same neutralizing antigenic sites as those of the native virus.

2) Evaluation of antigenicity by indirect ELISA assay

Various swine vesicular disease virus monoclonal antibodies were coated onto ELISA plates at 4°C overnight. Then, to the ELISA plates, each of the inactivated swine vesicular virus and the inventive swine vesicular virus-similar particle was added and then allowed to react with the antibodies at 37°C for 1 hour. Following washing step using PBST, peroxidase-conjugated standard monoclonal antibody 5B7 was added thereto and allowed to react at 37 °C for 1 hour. Following washing step using PBST, an ortho-phenylenediamine (OPD) solution as a color-developing agent was added thereto and allowed to react for 10 minutes, and the reactions were terminated with a sulfuric acid solution. Next, the reaction solutions were measured for the absorbance at 492 nm, and the swine vesicular disease virus and the swine vesicular disease virus-like particle were detected. In this regard, the reactivities of each antigen to various monoclonal antibodies were comparatively analyzed by the measurement of absorbance, and the results are shown in Table 2 below.

[Table 2]

| Monoclonal Antibodies | Indirect ELISA (absorbance) | |
|---|---|---|
| | Inventive virus-like particle | Swine vesicular disease virus |
| A | 0.14 | 0.3 |
| B | 0.14 | 0.22 |
| C | 0.07 | 0.08 |
| D | 0.1 | 0.12 |
| E | 0.12 | 0.13 |
| F | 0.07 | 0.06 |
| G | 0.52 | 0.55 |
| H | 1.84 | 2.06 |
| I | 1.1 | 1.38 |
| J | 0.6 | 0.66 |
| K | 0.09 | 0.07 |

(continued)

| Monoclonal Antibodies | Indirect ELISA (absorbance) | |
|---|---|---|
| | Inventive virus-like particle | Swine vesicular disease virus |
| L | 0.11 | 0.1 |
| ※ Reactivity correlation coefficient between two antigens: 0.995 (99.5%) | | |

As can be seen in Table 2 above, the comparison of reactivity between the two antigens showed that the correlation coefficient between the inventive swine vesicular disease virus-like particle and the inactivated swine vesicular disease virus antigen was 0.995, indicating that the antigenicities of the two antigens are significant ly similar to each other.

The above results indicate that the inventive virus-like particle can substitute for the prior inactivated virus antigen as a diagnostic antigen in the ELISA assay.

From the above results, it was found that the inventive swine vesicular disease virus-like particle has a structure highly similar to that of the native swine vesicular disease virus, and the use of the inventive virus-like particle allows the development of gene recombinant vaccines in the development of swine vesicular disease virus vaccines which are not yet commercialized.

[Industrial Applicability]

As can be seen from Examples above, the inventive method for preparing the inventive virus-like particle using one recombinant baculovirus inserted with both the P1 and 3CD genes of swine vesicular disease virus was first introduced also in the case of various viruses belonging to the Enterovirus genus.

Because the inventive swine vesicular disease virus-like particle has a structure and antigenicity highly similar to those of the native swine vesicular disease virus, it can be applied in various fields associated with the swine vesicular virus.

In terms of disease diagnosis, the swine vesicular disease virus-like particle according to the present invention can substitute for the prior inactivated swine vesicular disease virus antigen which has been used as a diagnostic antigen in a kit for the detection of swine vesicular disease antibodies. Thus, the inventive virus-like particle has an advantage in that it is prepared without a hazardous and complicated operation where live virus must be handled to prepare the inactivated virus antigen. Also, the inventive virus-like particle is highly useful as a diagnostic antigen because it can be produced in a general laboratory in a safe and easy manner.

In terms of the prevention of disease, the inventive swine vesicular disease virus-like particle prepared by the gene recombination technique can be developed into a preventive subunit vaccine because it has an antigenicity similar to that of the native swine vesicular disease virus. Also, because the inventive virus-like particle can be easily produced in a laboratory by gene recombination, it allows the rapid and safe production of a preventive vaccine in case of emergency. Also, because a preventive vaccine against the swine vesicular disease virus has not been yet developed, the inventive swine vesicular disease virus-like particle shows a possibility for the development of a first gene recombinant vaccine.

As can be seen from the foregoing, the inventive method for preparing the virus-like particle may likewise be applied in the case of other various viruses, members of the Enterovirus genus, which have the same genetic structure as that of the swine vesicular disease virus. Because the inventive method is a preparation method overcoming limitations shown in the preparation of virus-like particles in other enteroviruses, such as poliovirus, it may likewise be applied for other various enteroviruses, in addition to the swine vesicular disease virus shown in Examples. Accordingly, it is expected that the inventive method will allow the development of either diagnostic antigens for various enterovirus diseases or gene recombinant vaccines.

## Claims

1. A baculovirus expression vector containing swine vesicular disease virus genes, wherein said swine vesicular disease virus genes consist of the structural protein precursor P1 gene of SEQ ID NO: 1 and 3CD protease gene of SEQ ID NO: 3.

2. A recombinant baculovirus transformed with the baculovirus expression vector of Claim 1.

3. Insect cells transfected with the recombinant baculovirus of Claim 2.

4. The insect cells of Claim 3, wherein the insect cells are *Spodoptera* frugiperda.

5. A recombinant baculovirus produced by the insect cells of Claim 3 or 4, wherein said recombinant baculovirus contains swine vesicular disease virus genes consisting of the structural protein precursor P1 gene of SEQ ID NO: 1 and 3CD protease gene of SEQ ID NO: 3.

6. A swine vesicular disease virus-like particle prepared using the recombinant baculovirus of Claim 5, wherein said virus-like particle is formed from individual proteins obtained from structural protein precursor P1, wherein the structural protein precursor P1 is expressed by the structural protein precursor P1 gene of SEQ ID NO: 1, and then digested by 3CD protease expressed by 3CD protease gene of SEQ ID NO:3.

7. A method for preparing a swine vesicular disease virus-like particle, the method comprising the steps of:

   (1) amplifying the structural protein precursor P1 gene and 3CD protease gene of swine vesicular disease virus by polymerase chain reaction (PCR);

(2) inserting the P1 gene into a baculovirus expression vector pFastBacDual so as to prepare pFastBacDual/P1;

(3) inserting the 3CD gene into the pFastBacDual/P1 inserted with the P1 gene so as to prepare pFastBacDual/P1/3CD;

(4) transforming a cell (DH10Bac) containing a baculovirus shuttle vector Bacmid with the pFastBacDual/P1/3CD containing the P1 gene and the 3CD gene so as to prepare recombinant baculovirus gene Bacmid/P1/3CD;

(5) transfecting insect cells with the Bacmid/P1/3CD gene;

(6) subjecting the supernatant of the transfected insect cells to a plaque assay so as to isolate a pure recombinant baculovirus; and

(7) infecting insect cells with the recombinant baculovirus so as to obtain a swine vesicular disease virus-like particle.

## Patentansprüche

1. Baculovirus-Expressionsvektor, beinhaltend Virusgene der vesikulären Schweinekrankheit, wobei die Virusgene der vesikulären Schweinekrankheit aus dem strukturellen Proteinvorläufer-P1-Gen von SEQ ID NO: 1 und dem 3CD-Protease-Gen von SEQ ID NO: 3 bestehen.

2. Rekombinanter Baculovirus, transformiert mit dem Baculovirus-Expressionsvektor nach Anspruch 1.

3. Insektenzellen, transfiziert mit dem rekombinanten Baculovirus nach Anspruch 2.

4. Insektenzellen nach Anspruch 3, wobei die Insektenzellen *Spodoptera frugiperda* sind.

5. Rekombinanter Baculovirus, hergestellt durch die Insektenzellen nach Anspruch 3 oder 4, wobei der rekombinante Baculovirus Virusgene der vesikulären Schweinekrankheit enthält, die aus dem strukturellen Proteinvorläufer-P1-Gen von SEQ ID NO: 1 und dem 3CD-Protease-Gen von SEQ ID NO: 3 bestehen.

6. Partikel ähnlich zum vesikulären Schweinekrankheitsvirus, das unter Verwendung des rekombinanten Baculovirus nach Anspruch 5 bereitgestellt ist, wobei das virusähnliche Partikel aus individuellen Proteinen gebildet ist, die aus dem strukturellen Proteinvorläufer P1 erhalten sind, wobei der strukturelle Proteinvorläufer P1 durch das strukturelle Proteinvorläufer-P1-Gen von SEQ ID NO: 1 exprimiert ist und dann durch 3CD-Protease verdaut ist, welche durch das 3CD-Protease-Gen von SEQ ID NO: 3 exprimiert ist.

7. Verfahren zum Bereitstellen eines Partikels ähnlich zum vesikulären Schweinekrankheitsvirus, wobei das Verfahren die Schritte umfasst:

(1) Amplifizieren des strukturellen Proteinvorläufer-P1-Gens und des 3CD-Protease-Gens der vesikulären Schweinekrankheit durch Polymerase-Kettenreaktion (PCR);

(2) Einfügen des P1-Gens in einen Baculovirus-Expressionsvektor pFastBacDual, um pFastBacDual/P1 bereitzustellen;

(3) Einfügen des 3CD-Gens in den mit dem P1-Gen eingefügten pFastBacDual/P1, um pFastBacDual/P1/3CD bereitzustellen;

(4) Transformieren einer Zelle (DH10Bac), beinhaltend einen Baculovirus-Shuttle-Vektor Bacmid, mit dem pFastBacDual/P1/3CD, beinhaltend das P1-Gen und das 3CD-Gen, um ein rekombinantes Baculovirus-Gen Bacmid/P1/3CD bereitzustellen;

(5) Transfizieren von Insektenzellen mit dem Bacmid/P1/3CD-Gen;

(6) Einbringen des Überstands der transfizierten Insektenzellen in einen Plaque-Assay, um einen reinen rekombinanten Baculovirus zu isolieren; und

(7) Infizieren von Insektenzellen mit dem rekombinanten Baculovirus, um ein Partikel ähnlich zum vesikulären Schweinekrankheitsvirus zu erhalten.

## Revendications

1. Vecteur d'expression de baculovirus contenant des gènes du virus de la maladie vésiculeuse du porc, dans lequel lesdits gènes du virus de la maladie vésiculeuse du porc se composent du gène P1 précurseur de protéine structurale de SEQ ID NO:1 et du gène de la protéase 3CD de SEQ ID NO: 3.

**EP 1 802 759 B1**

2. Baculovirus recombinant transformé avec le vecteur d'expression de baculovirus de la revendication 1.

3. Cellules d'insecte transfectées avec le baculovirus recombinant de la revendication 2.

4. Cellules d'insecte selon la revendication 3, dans lesquelles les cellules d'insecte sont *Spodoptera frugiperda.*

5. Baculovirus recombinant produit par les cellules d'insecte de la revendication 3 ou 4, dans lequel ledit baculovirus recombinant contient des gènes du virus de la maladie vésiculeuse du porc constitué du gène P1 précurseur de protéine structurale de SEQ ID NO:1 et du gène de la protéase 3CD de SEQ ID NO:3.

6. Particule pseudo-virale de la maladie vésiculeuse du porc préparée à l'aide du baculovirus recombinant de la revendication 5, dans laquelle ladite particule pseudo-virale est formée à partir de protéines individuelles obtenues à partir du précurseur P1 de protéine structurale, dans laquelle le précurseur P1 de protéine structurale est exprimé par le gène P1 précurseur de protéine structurale de SEQ ID NO: 1, puis digéré par la protéase 3CD exprimée par le gène de la protéase 3CD de SEQ ID NO: 3.

7. Procédé de préparation d'une particule pseudo-virale de la maladie vésiculeuse du porc, le procédé comprenant les étapes :

(1) d'amplification du gène P1 précurseur de protéine structurale et du gène de la protéase 3CD du virus de la maladie vésiculeuse du porc par une amplification en chaîne par polymérase (PCR) ;
(2) d'insertion du gène P1 dans un vecteur d'expression de baculovirus pFastBacDual de manière à préparer pFastBacDual/P1;
(3) d'insertion du gène 3CD dans le pFastBacDual/P1 inséré avec le gène P1 de manière à préparer pFastBacDual/P1/3CD ;
(4) de transformation d'une cellule (DH10Bac contenant un vecteur navette de baculovirus Bacmid avec le pFastBacDual/P1/3CD contenant le gène P1 et le gène 3CD de manière à préparer le gène de baculovirus recombinant Bacmid/P1/3CD ;
(5) de transfection de cellules d'insecte avec le gène Bacmid/P1/3CD ;
(6) de soumission du surnageant des cellules d'insecte transfectées à une méthode des plages de lyse de manière à isoler un baculovirus recombinant pur; et
(7) d'infection de cellules d'insecte avec le baculovirus recombinant de manière à obtenir une particule pseudo-virale de la maladie vésiculeuse du porc.

**29**

【Figure 1】

【Figure 2】

【Figure 3】

【Figure 4】

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 200483994

- KR 2005003274 W

**Non-patent literature cited in the description**

- *Journal of Virological Methods,* 1995, vol. 52, 155-167
- *Virus Research,* 1998, vol. 57, 163-170

- *Journal of General Virology,* 1989, vol. 70, 1453-1463
- *Virology,* 1993, vol. 192, 512-524
- *Biotechnology Letters,* 2003, vol. 25, 919-925

SEQUENCE LISTING

<110> Republic of Korea (Management : Ministry of Agriculture and Forestry, National Veterinary Research)

<120> Construct of swine vesicular disease virus like particle using genetic engineering and the method for manufacturing thereof

<130> SR 30989 SG

<140> EP 07XXXXXX
<141> 2005-10-05

<150> KR 2004-83994
<151> 2004-10-20

<150> PCT/KR2005/003274
<151> 2005-10-05

<160> 8

<170> Patent In version 3.1

<210> 1
<211> 2553
<212> DNA
<213> Swine vesicular disease virus

<220>
<221> CDS
<222> (1)..(2553)
<223>

<400> 1

```
atg gga gct caa gtg tca aca caa aag acc ggt gct cat gag acc agc      48
Met Gly Ala Gln Val Ser Thr Gln Lys Thr Gly Ala His Glu Thr Ser
1               5                   10                  15

ttg agt gca gcg ggc aac tca gtc att cat tac aca aac ata aac tac      96
Leu Ser Ala Ala Gly Asn Ser Val Ile His Tyr Thr Asn Ile Asn Tyr
                20                  25                  30

tac aag gat gct gct tcg aat tca gca aat aga caa gac ttc aca cag     144
Tyr Lys Asp Ala Ala Ser Asn Ser Ala Asn Arg Gln Asp Phe Thr Gln
            35                  40                  45

gac ccg ggg aag ttc acc gaa cct gtg aaa gac atc atg gtc aaa tcc     192
Asp Pro Gly Lys Phe Thr Glu Pro Val Lys Asp Ile Met Val Lys Ser
        50                  55                  60

atg cct gct ctc aat tcc cca tca gca gag gag tgt ggc tac agt gac     240
Met Pro Ala Leu Asn Ser Pro Ser Ala Glu Glu Cys Gly Tyr Ser Asp
65                  70                  75                  80

agg gta aga tcc atc acc tta ggg aat tcg acc ata aca act caa gaa     288
Arg Val Arg Ser Ile Thr Leu Gly Asn Ser Thr Ile Thr Thr Gln Glu
                85                  90                  95

tgt gca aac gtg gta gtt gga tat ggg gtg tgg cca act tac ttg aag     336
Cys Ala Asn Val Val Val Gly Tyr Gly Val Trp Pro Thr Tyr Leu Lys
            100                 105                 110
```

```
gat gaa gag gca aca gca gag gat caa ccc act caa cca gat gtg gcc    384
Asp Glu Glu Ala Thr Ala Glu Asp Gln Pro Thr Gln Pro Asp Val Ala
        115                 120                 125

acg tgc agg ttt tac acg ctc gaa tcc gtg atg tgg caa cag agt tca    432
Thr Cys Arg Phe Tyr Thr Leu Glu Ser Val Met Trp Gln Gln Ser Ser
    130                 135                 140

cca ggc tgg tgg tgg aag ttc cct gac gcg ttg tcc aac atg ggg cta    480
Pro Gly Trp Trp Trp Lys Phe Pro Asp Ala Leu Ser Asn Met Gly Leu
145                 150                 155                 160

ttt ggg caa aat atg cag tac cac tac ctt ggg aga gcc gga tac acg    528
Phe Gly Gln Asn Met Gln Tyr His Tyr Leu Gly Arg Ala Gly Tyr Thr
                165                 170                 175

ata cac gtg cag tgc aac gcg tcc aaa ttt cac caa ggg tgt ctg ctg    576
Ile His Val Gln Cys Asn Ala Ser Lys Phe His Gln Gly Cys Leu Leu
                180                 185                 190

gtg gta tgt gtg cca gaa gca gag atg ggg tgt gcc acg ttg gcc aat    624
Val Val Cys Val Pro Glu Ala Glu Met Gly Cys Ala Thr Leu Ala Asn
            195                 200                 205

aag cct gac cca aaa agc ctg agt aaa ggg gaa ata gcc aac atg ttt    672
Lys Pro Asp Pro Lys Ser Leu Ser Lys Gly Glu Ile Ala Asn Met Phe
    210                 215                 220

gaa tcc caa aac tcc acc ggg gaa acg gcc gtg caa gct aat gtg atc    720
Glu Ser Gln Asn Ser Thr Gly Glu Thr Ala Val Gln Ala Asn Val Ile
225                 230                 235                 240

aat gct ggc atg ggt gtt ggt gtt ggt aat cta act atc ttc ccc cat    768
Asn Ala Gly Met Gly Val Gly Val Gly Asn Leu Thr Ile Phe Pro His
                245                 250                 255

cag tgg atc aac ttg cgc act aac aac agc gct acg att gtc atg cca    816
Gln Trp Ile Asn Leu Arg Thr Asn Asn Ser Ala Thr Ile Val Met Pro
                260                 265                 270

tat ata aac agc gtg ccc atg gac aac atg ttc aga cac aac aat ttt    864
Tyr Ile Asn Ser Val Pro Met Asp Asn Met Phe Arg His Asn Asn Phe
                275                 280                 285

aca ctc atg gtc atc ccg ttc gcc cca ctg agc tac agc aca ggg gct    912
Thr Leu Met Val Ile Pro Phe Ala Pro Leu Ser Tyr Ser Thr Gly Ala
    290                 295                 300

acc acg tac gta cca atc act gta aca gtg gcg cca atg tgc gct gaa    960
Thr Thr Tyr Val Pro Ile Thr Val Thr Val Ala Pro Met Cys Ala Glu
305                 310                 315                 320

tat aat ggg ctg cgt ctg gcc ggc aag caa ggt tta cca acg ctg tcg   1008
Tyr Asn Gly Leu Arg Leu Ala Gly Lys Gln Gly Leu Pro Thr Leu Ser
                325                 330                 335

aca ccc ggg agc aac cag ttt ctc acg tcc gat gac ttc cag tca cca   1056
Thr Pro Gly Ser Asn Gln Phe Leu Thr Ser Asp Asp Phe Gln Ser Pro
                340                 345                 350
```

```
tca gcc atg cca caa ttc gat gtc act cct gag atg gat att cca gga     1104
Ser Ala Met Pro Gln Phe Asp Val Thr Pro Glu Met Asp Ile Pro Gly
        355             360             365

caa gtc aac aac ttg atg gag att gca gaa gta gat tct gta gtg cct     1152
Gln Val Asn Asn Leu Met Glu Ile Ala Glu Val Asp Ser Val Val Pro
        370             375             380

gta aat aac aca gaa ggg aaa gtg atg tca att gag gca tac cag ata     1200
Val Asn Asn Thr Glu Gly Lys Val Met Ser Ile Glu Ala Tyr Gln Ile
385             390             395             400

cct gtg caa tcg aat cca acc aac ggt tct cag gtt ttt ggg ttc cca     1248
Pro Val Gln Ser Asn Pro Thr Asn Gly Ser Gln Val Phe Gly Phe Pro
            405             410             415

ttg acc cca ggg gcc aat agt gtg tta aac agg act ttg ctg gga gaa     1296
Leu Thr Pro Gly Ala Asn Ser Val Leu Asn Arg Thr Leu Leu Gly Glu
            420             425             430

atc tta aac tac tat gcc cat tgg tca ggc agc atc aaa cta aca ttt     1344
Ile Leu Asn Tyr Tyr Ala His Trp Ser Gly Ser Ile Lys Leu Thr Phe
        435             440             445

atg ttt tgc ggg tca gcg atg gct aca gga aaa ttc tta ctg gcg tac     1392
Met Phe Cys Gly Ser Ala Met Ala Thr Gly Lys Phe Leu Leu Ala Tyr
    450             455             460

tca cca ccg gga gct ggg gca ccg acc aca cgc aag gag gcg atg cta     1440
Ser Pro Pro Gly Ala Gly Ala Pro Thr Thr Arg Lys Glu Ala Met Leu
465             470             475             480

ggt act cac gtg atc tgg gat gtg ggt cta caa tcg agc tgc gta ttg     1488
Gly Thr His Val Ile Trp Asp Val Gly Leu Gln Ser Ser Cys Val Leu
            485             490             495

tgt ata cca tgg att agt caa acg cac tac agg tat gta gta atg gat     1536
Cys Ile Pro Trp Ile Ser Gln Thr His Tyr Arg Tyr Val Val Met Asp
        500             505             510

gaa tac acc gct ggt gga tac ata act tgc tgg tat caa aca aat att     1584
Glu Tyr Thr Ala Gly Gly Tyr Ile Thr Cys Trp Tyr Gln Thr Asn Ile
        515             520             525

gtg gtg cct gca gat gca cag agt gac tgt aag atc ttg tgt ttt gtg     1632
Val Val Pro Ala Asp Ala Gln Ser Asp Cys Lys Ile Leu Cys Phe Val
        530             535             540

tcg gca tgt aac gat ttt tca gtt agg atg ctc aag gac aca ccc ttt     1680
Ser Ala Cys Asn Asp Phe Ser Val Arg Met Leu Lys Asp Thr Pro Phe
545             550             555             560

ata aaa cag gat aat ttc ttc caa ggg ccc cca gga gag gtg atg gga     1728
Ile Lys Gln Asp Asn Phe Phe Gln Gly Pro Pro Gly Glu Val Met Gly
            565             570             575

aga gcc att gcc cgc gtc gct gat acc att ggg agc gga cca gtt aac     1776
Arg Ala Ile Ala Arg Val Ala Asp Thr Ile Gly Ser Gly Pro Val Asn
            580             585             590

tcg gaa tcc att cca gcc cta acc gcc gca gag aca ggg cac acg tca     1824
```

13

```
Ser Glu Ser Ile Pro Ala Leu Thr Ala Ala Glu Thr Gly His Thr Ser
        595                 600                 605

caa gtt gta cca tca gac aca atg caa act aga cac gtg aag aat tac      1872
Gln Val Val Pro Ser Asp Thr Met Gln Thr Arg His Val Lys Asn Tyr
        610                 615                 620

cat tca aga tca gag tcg aca gtg gag aac ttc ctg tgc aga tct gca      1920
His Ser Arg Ser Glu Ser Thr Val Glu Asn Phe Leu Cys Arg Ser Ala
625                 630                 635                 640

tgc gtc ttc tac acc aca tac aag aac cat gac tcc gat ggc gac aac      1968
Cys Val Phe Tyr Thr Thr Tyr Lys Asn His Asp Ser Asp Gly Asp Asn
                645                 650                 655

ttc gcc tac tgg gtg atc aac aca cgg caa gtt gct caa ctg cgt cgg      2016
Phe Ala Tyr Trp Val Ile Asn Thr Arg Gln Val Ala Gln Leu Arg Arg
                660                 665                 670

aag ctc gaa atg ttc acg tac gca aga ttt gat ctg gag ttg acc ttc      2064
Lys Leu Glu Met Phe Thr Tyr Ala Arg Phe Asp Leu Glu Leu Thr Phe
        675                 680                 685

gtg atc act agc act cag gaa caa ccc acc gtt cga ggt caa gat gca      2112
Val Ile Thr Ser Thr Gln Glu Gln Pro Thr Val Arg Gly Gln Asp Ala
        690                 695                 700

cca gtg ctc acc cac caa ata atg tat gta cct cca ggt ggt cca gta      2160
Pro Val Leu Thr His Gln Ile Met Tyr Val Pro Pro Gly Gly Pro Val
705                 710                 715                 720

ccc aca aag gta aac agc tac agc tgg caa acg tcc acc aac ccg agt      2208
Pro Thr Lys Val Asn Ser Tyr Ser Trp Gln Thr Ser Thr Asn Pro Ser
                725                 730                 735

gtg ttc tgg acg gaa ggg agc gca ccg cct cga atg tcg ata cca ttc      2256
Val Phe Trp Thr Glu Gly Ser Ala Pro Pro Arg Met Ser Ile Pro Phe
                740                 745                 750

att ggc ata ggc aac gca tac agc atg ttc tat gac ggg tgg gcc agg      2304
Ile Gly Ile Gly Asn Ala Tyr Ser Met Phe Tyr Asp Gly Trp Ala Arg
                755                 760                 765

ttt gac aag caa ggg aca tac ggc atc agc aca tta aac aac atg ggg      2352
Phe Asp Lys Gln Gly Thr Tyr Gly Ile Ser Thr Leu Asn Asn Met Gly
        770                 775                 780

aca cta tat atg aga cat gtg aat gat ggg ggt ccc ggt ccc att gta      2400
Thr Leu Tyr Met Arg His Val Asn Asp Gly Gly Pro Gly Pro Ile Val
785                 790                 795                 800

agc aca gta cga att tac ttc aag cca aag cac gtc aaa acg tgg gtc      2448
Ser Thr Val Arg Ile Tyr Phe Lys Pro Lys His Val Lys Thr Trp Val
                805                 810                 815

cca aga ccg ccc aga cta tgt caa tac caa aag gct ggc aac gtg aat      2496
Pro Arg Pro Pro Arg Leu Cys Gln Tyr Gln Lys Ala Gly Asn Val Asn
                820                 825                 830

ttt gaa ccc act ggt gtg act gag ggt agg aca gat ata aca acc atg      2544
Phe Glu Pro Thr Gly Val Thr Glu Gly Arg Thr Asp Ile Thr Thr Met
```

14

835     840     845

```
aaa acc act                                                    2553
Lys Thr Thr
    850
```

<210> 2
<211> 851
<212> PRT
<213> Swine vesicular disease virus

<400> 2

```
Met Gly Ala Gln Val Ser Thr Gln Lys Thr Gly Ala His Glu Thr Ser
1               5                   10                  15


Leu Ser Ala Ala Gly Asn Ser Val Ile His Tyr Thr Asn Ile Asn Tyr
            20                  25                  30


Tyr Lys Asp Ala Ala Ser Asn Ser Ala Asn Arg Gln Asp Phe Thr Gln
        35                  40                  45


Asp Pro Gly Lys Phe Thr Glu Pro Val Lys Asp Ile Met Val Lys Ser
    50                  55                  60


Met Pro Ala Leu Asn Ser Pro Ser Ala Glu Glu Cys Gly Tyr Ser Asp
65                  70                  75                  80


Arg Val Arg Ser Ile Thr Leu Gly Asn Ser Thr Ile Thr Thr Gln Glu
                85                  90                  95


Cys Ala Asn Val Val Val Gly Tyr Gly Val Trp Pro Thr Tyr Leu Lys
            100                 105                 110


Asp Glu Glu Ala Thr Ala Glu Asp Gln Pro Thr Gln Pro Asp Val Ala
            115                 120                 125


Thr Cys Arg Phe Tyr Thr Leu Glu Ser Val Met Trp Gln Gln Ser Ser
    130                 135                 140


Pro Gly Trp Trp Trp Lys Phe Pro Asp Ala Leu Ser Asn Met Gly Leu
145                 150                 155                 160


Phe Gly Gln Asn Met Gln Tyr His Tyr Leu Gly Arg Ala Gly Tyr Thr
                165                 170                 175


Ile His Val Gln Cys Asn Ala Ser Lys Phe His Gln Gly Cys Leu Leu
            180                 185                 190
```

```
Val Val Cys Val Pro Glu Ala Glu Met Gly Cys Ala Thr Leu Ala Asn
    195             200             205

Lys Pro Asp Pro Lys Ser Leu Ser Lys Gly Glu Ile Ala Asn Met Phe
    210             215             220

Glu Ser Gln Asn Ser Thr Gly Glu Thr Ala Val Gln Ala Asn Val Ile
225             230             235             240

Asn Ala Gly Met Gly Val Gly Val Gly Asn Leu Thr Ile Phe Pro His
            245             250             255

Gln Trp Ile Asn Leu Arg Thr Asn Asn Ser Ala Thr Ile Val Met Pro
            260             265             270

Tyr Ile Asn Ser Val Pro Met Asp Asn Met Phe Arg His Asn Asn Phe
            275             280             285

Thr Leu Met Val Ile Pro Phe Ala Pro Leu Ser Tyr Ser Thr Gly Ala
    290             295             300

Thr Thr Tyr Val Pro Ile Thr Val Thr Val Ala Pro Met Cys Ala Glu
305             310             315             320

Tyr Asn Gly Leu Arg Leu Ala Gly Lys Gln Gly Leu Pro Thr Leu Ser
            325             330             335

Thr Pro Gly Ser Asn Gln Phe Leu Thr Ser Asp Asp Phe Gln Ser Pro
            340             345             350

Ser Ala Met Pro Gln Phe Asp Val Thr Pro Glu Met Asp Ile Pro Gly
            355             360             365

Gln Val Asn Asn Leu Met Glu Ile Ala Glu Val Asp Ser Val Val Pro
    370             375             380

Val Asn Asn Thr Glu Gly Lys Val Met Ser Ile Glu Ala Tyr Gln Ile
385             390             395             400

Pro Val Gln Ser Asn Pro Thr Asn Gly Ser Gln Val Phe Gly Phe Pro
            405             410             415

Leu Thr Pro Gly Ala Asn Ser Val Leu Asn Arg Thr Leu Leu Gly Glu
            420             425             430
```

Ile Leu Asn Tyr Tyr Ala His Trp Ser Gly Ser Ile Lys Leu Thr Phe
435 440 445

Met Phe Cys Gly Ser Ala Met Ala Thr Gly Lys Phe Leu Leu Ala Tyr
450 455 460

Ser Pro Pro Gly Ala Gly Ala Pro Thr Thr Arg Lys Glu Ala Met Leu
465 470 475 480

Gly Thr His Val Ile Trp Asp Val Gly Leu Gln Ser Ser Cys Val Leu
485 490 495

Cys Ile Pro Trp Ile Ser Gln Thr His Tyr Arg Tyr Val Val Met Asp
500 505 510

Glu Tyr Thr Ala Gly Gly Tyr Ile Thr Cys Trp Tyr Gln Thr Asn Ile
515 520 525

Val Val Pro Ala Asp Ala Gln Ser Asp Cys Lys Ile Leu Cys Phe Val
530 535 540

Ser Ala Cys Asn Asp Phe Ser Val Arg Met Leu Lys Asp Thr Pro Phe
545 550 555 560

Ile Lys Gln Asp Asn Phe Phe Gln Gly Pro Pro Gly Glu Val Met Gly
565 570 575

Arg Ala Ile Ala Arg Val Ala Asp Thr Ile Gly Ser Gly Pro Val Asn
580 585 590

Ser Glu Ser Ile Pro Ala Leu Thr Ala Ala Glu Thr Gly His Thr Ser
595 600 605

Gln Val Val Pro Ser Asp Thr Met Gln Thr Arg His Val Lys Asn Tyr
610 615 620

His Ser Arg Ser Glu Ser Thr Val Glu Asn Phe Leu Cys Arg Ser Ala
625 630 635 640

Cys Val Phe Tyr Thr Thr Tyr Lys Asn His Asp Ser Asp Gly Asp Asn
645 650 655

Phe Ala Tyr Trp Val Ile Asn Thr Arg Gln Val Ala Gln Leu Arg Arg
660 665 670

Lys Leu Glu Met Phe Thr Tyr Ala Arg Phe Asp Leu Glu Leu Thr Phe

```
                    675                     680                     685

        Val Ile Thr Ser Thr Gln Glu Gln Pro Thr Val Arg Gly Gln Asp Ala
            690                 695                 700

        Pro Val Leu Thr His Gln Ile Met Tyr Val Pro Pro Gly Gly Pro Val
        705                 710                 715                 720

        Pro Thr Lys Val Asn Ser Tyr Ser Trp Gln Thr Ser Thr Asn Pro Ser
                        725                 730                 735

        Val Phe Trp Thr Glu Gly Ser Ala Pro Pro Arg Met Ser Ile Pro Phe
                740                 745                 750

        Ile Gly Ile Gly Asn Ala Tyr Ser Met Phe Tyr Asp Gly Trp Ala Arg
                755                 760                 765

        Phe Asp Lys Gln Gly Thr Tyr Gly Ile Ser Thr Leu Asn Asn Met Gly
            770                 775                 780

        Thr Leu Tyr Met Arg His Val Asn Asp Gly Gly Pro Gly Pro Ile Val
        785                 790                 795                 800

        Ser Thr Val Arg Ile Tyr Phe Lys Pro Lys His Val Lys Thr Trp Val
                        805                 810                 815

        Pro Arg Pro Pro Arg Leu Cys Gln Tyr Gln Lys Ala Gly Asn Val Asn
                820                 825                 830

        Phe Glu Pro Thr Gly Val Thr Glu Gly Arg Thr Asp Ile Thr Thr Met
                835                 840                 845

        Lys Thr Thr
            850
```

```
<210> 3
<211> 1935
<212> DNA
<213> Swine vesicular disease virus

<220>
<221> CDS
<222> (1)..(1935)
<223>

<400> 3
```

```
ggt cca gcg ttt gag ttc gcc gtg gcg atg atg aaa aga aac gcc agt      48
Gly Pro Ala Phe Glu Phe Ala Val Ala Met Met Lys Arg Asn Ala Ser
1               5                   10                  15
```

```
aca gtg aaa act gag tac ggt gaa ttc acc atg ctt ggg att tac gac          96
Thr Val Lys Thr Glu Tyr Gly Glu Phe Thr Met Leu Gly Ile Tyr Asp
        20                  25                  30

agg tgg gcg gtg ttg cca cgc cat gcc aaa cct ggc ccc acc atc ttg         144
Arg Trp Ala Val Leu Pro Arg His Ala Lys Pro Gly Pro Thr Ile Leu
        35                  40                  45

atg aac gac cag gta gtc gga gtg ttg gac gcc aag gaa cta gtt gat         192
Met Asn Asp Gln Val Val Gly Val Leu Asp Ala Lys Glu Leu Val Asp
        50                  55                  60

aaa gat ggg acc aac ctg gaa ttg act ctc ttg aag ctc aac cgc aac         240
Lys Asp Gly Thr Asn Leu Glu Leu Thr Leu Leu Lys Leu Asn Arg Asn
65                  70                  75                  80

gag aag ttt aga gac atc agg gga ttc tta gca cga gag gag gtc gaa         288
Glu Lys Phe Arg Asp Ile Arg Gly Phe Leu Ala Arg Glu Glu Val Glu
                    85                  90                  95

gtg aac gaa gct gtc cta gca ata aac aca agt aaa ttc ccg aat atg         336
Val Asn Glu Ala Val Leu Ala Ile Asn Thr Ser Lys Phe Pro Asn Met
                100                 105                 110

tac ata ccc gtg ggc cgg gta acc gac tat ggg ttc tta aat ctg ggt         384
Tyr Ile Pro Val Gly Arg Val Thr Asp Tyr Gly Phe Leu Asn Leu Gly
            115                 120                 125

gga acc ccc acg aag aga atg ctc atg tac aat ttc cca act agg gca         432
Gly Thr Pro Thr Lys Arg Met Leu Met Tyr Asn Phe Pro Thr Arg Ala
            130                 135                 140

ggc cag tgt ggg ggt gtc ctt atg tca aca ggg aaa gtc ctg gga ata         480
Gly Gln Cys Gly Gly Val Leu Met Ser Thr Gly Lys Val Leu Gly Ile
145                 150                 155                 160

cat gta gga ggg aat gga cac caa ggg ttt tca gcg gca ctc ctc aga         528
His Val Gly Gly Asn Gly His Gln Gly Phe Ser Ala Ala Leu Leu Arg
                165                 170                 175

cac tac ttc aac gag gag cag ggt gag ata gaa ttc att gag agc tcg         576
His Tyr Phe Asn Glu Glu Gln Gly Glu Ile Glu Phe Ile Glu Ser Ser
                180                 185                 190

aag gac gca gga ttt ccc gtg atc aac acc ccc agc aag aca aaa ttg         624
Lys Asp Ala Gly Phe Pro Val Ile Asn Thr Pro Ser Lys Thr Lys Leu
            195                 200                 205

gaa cca agt gtg ttt cac cac gtg ttc gag ggc aac aag gaa cca gcg         672
Glu Pro Ser Val Phe His His Val Phe Glu Gly Asn Lys Glu Pro Ala
            210                 215                 220

gtt ctc aga aat ggg gac cca cga ctc aag gcc aac ttt gag gag gca         720
Val Leu Arg Asn Gly Asp Pro Arg Leu Lys Ala Asn Phe Glu Glu Ala
225                 230                 235                 240

atc ttc tcc aag tac att ggc aat gtt aac aca cat gta gac gag tac         768
Ile Phe Ser Lys Tyr Ile Gly Asn Val Asn Thr His Val Asp Glu Tyr
                245                 250                 255
```

```
atg atg gag gct gta gat cat tat gca gga caa cta gcc aca ctg gac          816
Met Met Glu Ala Val Asp His Tyr Ala Gly Gln Leu Ala Thr Leu Asp
        260                 265                 270

atc agc acg gag ccc atg aag cta gaa gat gcc gtg tat ggc act gag          864
Ile Ser Thr Glu Pro Met Lys Leu Glu Asp Ala Val Tyr Gly Thr Glu
        275                 280                 285

ggg ctc gaa gca cta gac ctg acc acc agt gca ggt tac cct tat gtg          912
Gly Leu Glu Ala Leu Asp Leu Thr Thr Ser Ala Gly Tyr Pro Tyr Val
        290                 295                 300

gcc ctg ggt atc aag aaa aga gac atc cta tcc aag aag acc aga gac          960
Ala Leu Gly Ile Lys Lys Arg Asp Ile Leu Ser Lys Lys Thr Arg Asp
305                 310                 315                 320

ctt acc aag cta aag gaa tgc atg gac aaa tat ggt cta aac ctg cca         1008
Leu Thr Lys Leu Lys Glu Cys Met Asp Lys Tyr Gly Leu Asn Leu Pro
                    325                 330                 335

atg gta acc tat gtc aag gac gag ttg aga tct gcc gac aaa gtg gcc         1056
Met Val Thr Tyr Val Lys Asp Glu Leu Arg Ser Ala Asp Lys Val Ala
                    340                 345                 350

aag gga aag tcc agg ctc atc gag gct tct agc ctc aac gac tca gta         1104
Lys Gly Lys Ser Arg Leu Ile Glu Ala Ser Ser Leu Asn Asp Ser Val
        355                 360                 365

gca atg agg cag aca ttt gga aac cta tat aag act ttc cac ctc aac         1152
Ala Met Arg Gln Thr Phe Gly Asn Leu Tyr Lys Thr Phe His Leu Asn
        370                 375                 380

ccg ggc atc gtt acg ggt agc gcc gtt ggg tgt gac cca gat gtc ttt         1200
Pro Gly Ile Val Thr Gly Ser Ala Val Gly Cys Asp Pro Asp Val Phe
385                 390                 395                 400

tgg agc aag att ccc gtc atg ctc gat gga cat ctc ata gcg ttt gac         1248
Trp Ser Lys Ile Pro Val Met Leu Asp Gly His Leu Ile Ala Phe Asp
                    405                 410                 415

tat tca ggc tat gac gcc agc ctc agc cca gtg tgg ttt acg tgc ttg         1296
Tyr Ser Gly Tyr Asp Ala Ser Leu Ser Pro Val Trp Phe Thr Cys Leu
                    420                 425                 430

aaa ctc ctc ctg gag aag cta ggg tac aca aac aag gaa acg aac tac         1344
Lys Leu Leu Leu Glu Lys Leu Gly Tyr Thr Asn Lys Glu Thr Asn Tyr
        435                 440                 445

ata gac tac ctc tgt aat tcc cac cac ctg tac agg gac aaa cac tac         1392
Ile Asp Tyr Leu Cys Asn Ser His His Leu Tyr Arg Asp Lys His Tyr
        450                 455                 460

ttt gtg agg ggc ggc atg cca tca gga tgc tca ggc act agc ata ttt         1440
Phe Val Arg Gly Gly Met Pro Ser Gly Cys Ser Gly Thr Ser Ile Phe
465                 470                 475                 480

aat tcc atg att aac aac atc ata atc aga acc ctc atg ctg aag gtt         1488
Asn Ser Met Ile Asn Asn Ile Ile Ile Arg Thr Leu Met Leu Lys Val
                    485                 490                 495

tat aaa ggc att gat ttg gac caa ttc aga atg att gca tat ggg gat         1536
```

```
Tyr Lys Gly Ile Asp Leu Asp Gln Phe Arg Met Ile Ala Tyr Gly Asp
        500                 505                 510

gat gtg ata gct tca tac ccg tgg ccc atc gat gcc tca ctg cta gct   1584
Asp Val Ile Ala Ser Tyr Pro Trp Pro Ile Asp Ala Ser Leu Leu Ala
        515                 520                 525

gaa gca ggg aag gat tat ggc ttg atc atg acc cca gca gat aaa ggc   1632
Glu Ala Gly Lys Asp Tyr Gly Leu Ile Met Thr Pro Ala Asp Lys Gly
        530                 535                 540

gag tgt ttc aat gag gta acc tgg aca aac gtg acc ttc ctg aaa agg   1680
Glu Cys Phe Asn Glu Val Thr Trp Thr Asn Val Thr Phe Leu Lys Arg
545                 550                 555                 560

tac ttc agg gca gat gaa cag tac cca ttt ttg gtc cat cct gtc atg   1728
Tyr Phe Arg Ala Asp Glu Gln Tyr Pro Phe Leu Val His Pro Val Met
                565                 570                 575

cca atg aag gat ata cac gaa tcc att agg tgg act aaa gat cct aag   1776
Pro Met Lys Asp Ile His Glu Ser Ile Arg Trp Thr Lys Asp Pro Lys
                580                 585                 590

aac aca cag gat cac gtg cgc tcg ctg tgt cta ttg gcc tgg cac aac   1824
Asn Thr Gln Asp His Val Arg Ser Leu Cys Leu Leu Ala Trp His Asn
        595                 600                 605

ggg gag cac gaa tat gag gag ttt att cgt aag atc aga agc gtc cgc   1872
Gly Glu His Glu Tyr Glu Glu Phe Ile Arg Lys Ile Arg Ser Val Arg
        610                 615                 620

gta ggg cgc tgc ttg tcc ctc cct gcg ttt tca acg ctg cgc agg aag   1920
Val Gly Arg Cys Leu Ser Leu Pro Ala Phe Ser Thr Leu Arg Arg Lys
625                 630                 635                 640

tgg ttg gac tcc ttt                                               1935
Trp Leu Asp Ser Phe
                645
```

<210> 4
<211> 645
<212> PRT
<213> Swine vesicular disease virus

<400> 4

```
Gly Pro Ala Phe Glu Phe Ala Val Ala Met Met Lys Arg Asn Ala Ser
1                   5                   10                  15


Thr Val Lys Thr Glu Tyr Gly Glu Phe Thr Met Leu Gly Ile Tyr Asp
            20                  25                  30


Arg Trp Ala Val Leu Pro Arg His Ala Lys Pro Gly Pro Thr Ile Leu
        35                  40                  45


Met Asn Asp Gln Val Val Gly Val Leu Asp Ala Lys Glu Leu Val Asp
    50                  55                  60
```

Lys Asp Gly Thr Asn Leu Glu Leu Thr Leu Leu Lys Leu Asn Arg Asn
65                    70              75                    80

Glu Lys Phe Arg Asp Ile Arg Gly Phe Leu Ala Arg Glu Glu Val Glu
                85                    90                    95

Val Asn Glu Ala Val Leu Ala Ile Asn Thr Ser Lys Phe Pro Asn Met
                100               105               110

Tyr Ile Pro Val Gly Arg Val Thr Asp Tyr Gly Phe Leu Asn Leu Gly
          115               120               125

Gly Thr Pro Thr Lys Arg Met Leu Met Tyr Asn Phe Pro Thr Arg Ala
      130               135               140

Gly Gln Cys Gly Gly Val Leu Met Ser Thr Gly Lys Val Leu Gly Ile
145               150               155               160

His Val Gly Gly Asn Gly His Gln Gly Phe Ser Ala Ala Leu Leu Arg
                165               170               175

His Tyr Phe Asn Glu Glu Gln Gly Glu Ile Glu Phe Ile Glu Ser Ser
          180               185               190

Lys Asp Ala Gly Phe Pro Val Ile Asn Thr Pro Ser Lys Thr Lys Leu
      195               200               205

Glu Pro Ser Val Phe His His Val Phe Glu Gly Asn Lys Glu Pro Ala
      210               215               220

Val Leu Arg Asn Gly Asp Pro Arg Leu Lys Ala Asn Phe Glu Glu Ala
225               230               235               240

Ile Phe Ser Lys Tyr Ile Gly Asn Val Asn Thr His Val Asp Glu Tyr
                245               250               255

Met Met Glu Ala Val Asp His Tyr Ala Gly Gln Leu Ala Thr Leu Asp
                260               265               270

Ile Ser Thr Glu Pro Met Lys Leu Glu Asp Ala Val Tyr Gly Thr Glu
          275               280               285

Gly Leu Glu Ala Leu Asp Leu Thr Thr Ser Ala Gly Tyr Pro Tyr Val
      290               295               300

```
Ala Leu Gly Ile Lys Lys Arg Asp Ile Leu Ser Lys Lys Thr Arg Asp
305                 310             315                 320

Leu Thr Lys Leu Lys Glu Cys Met Asp Lys Tyr Gly Leu Asn Leu Pro
            325             330             335

Met Val Thr Tyr Val Lys Asp Glu Leu Arg Ser Ala Asp Lys Val Ala
            340             345             350

Lys Gly Lys Ser Arg Leu Ile Glu Ala Ser Ser Leu Asn Asp Ser Val
            355             360             365

Ala Met Arg Gln Thr Phe Gly Asn Leu Tyr Lys Thr Phe His Leu Asn
            370             375             380

Pro Gly Ile Val Thr Gly Ser Ala Val Gly Cys Asp Pro Asp Val Phe
385             390             395             400

Trp Ser Lys Ile Pro Val Met Leu Asp Gly His Leu Ile Ala Phe Asp
                405             410             415

Tyr Ser Gly Tyr Asp Ala Ser Leu Ser Pro Val Trp Phe Thr Cys Leu
            420             425             430

Lys Leu Leu Leu Glu Lys Leu Gly Tyr Thr Asn Lys Glu Thr Asn Tyr
            435             440             445

Ile Asp Tyr Leu Cys Asn Ser His His Leu Tyr Arg Asp Lys His Tyr
            450             455             460

Phe Val Arg Gly Gly Met Pro Ser Gly Cys Ser Gly Thr Ser Ile Phe
465             470             475             480

Asn Ser Met Ile Asn Asn Ile Ile Ile Arg Thr Leu Met Leu Lys Val
            485             490             495

Tyr Lys Gly Ile Asp Leu Asp Gln Phe Arg Met Ile Ala Tyr Gly Asp
            500             505             510

Asp Val Ile Ala Ser Tyr Pro Trp Pro Ile Asp Ala Ser Leu Leu Ala
            515             520             525

Glu Ala Gly Lys Asp Tyr Gly Leu Ile Met Thr Pro Ala Asp Lys Gly
            530             535             540
```

25

```
Glu Cys Phe Asn Glu Val Thr Trp Thr Asn Val Thr Phe Leu Lys Arg
545                 550                 555                 560

Tyr Phe Arg Ala Asp Glu Gln Tyr Pro Phe Leu Val His Pro Val Met
                565                 570                 575

Pro Met Lys Asp Ile His Glu Ser Ile Arg Trp Thr Lys Asp Pro Lys
                580                 585                 590

Asn Thr Gln Asp His Val Arg Ser Leu Cys Leu Leu Ala Trp His Asn
            595                 600                 605

Gly Glu His Glu Tyr Glu Glu Phe Ile Arg Lys Ile Arg Ser Val Arg
        610                 615                 620

Val Gly Arg Cys Leu Ser Leu Pro Ala Phe Ser Thr Leu Arg Arg Lys
625                 630                 635                 640

Trp Leu Asp Ser Phe
                645
```

<210> 5
<211> 26
<212> DNA
<213> Artificial

<220>
<223> Primer

<220>
<221> misc_feature
<222> (1)..(26)
<223> Forward primer for P1 gene

<400> 5
ggatccatgg gagctcaagt gtcaac          26

<210> 6
<211> 27
<212> DNA
<213> Artificial

<220>
<223> Primer

<220>
<221> misc_feature
<222> (1)..(27)
<223> Reverse primer for P1 gene

<400> 6
aagcttaagt ggttttcatg gttgtta          27

<210> 7
<211> 30
<212> DNA
<213> Artificial

<220>
<223> Primer

<220>
<221> misc_feature
<222> (1)..(30)
<223> Forward primer for 3CD gene

<400> 7
gccctcgaga tgggtccagc gtttgagttc        30

<210> 8
<211> 30
<212> DNA
<213> Artificial

<220>
<223> Primer

<220>
<221> misc_feature
<222> (1)..(30)
<223> Reverse primer for 3CD gene

<400> 8
gctcggtacc taaaaggagt ccaaccactt        30